# EUROPEAN PATENT APPLICATION

(11) **EP 4 775 244 A1**
(43) Date of publication of application: **15.07.2026**
(21) Application number: 26151046.5
(22) Date of filing: 09.01.2026
(51) Int. Cl.: A61M 16/06, A61M 16/04, A61M 16/08

(54) **NASAL CANNULAS INCLUDING MANIFOLDS HAVING NASAL PRONGS AND SYSTEMS INCLUDING SAME**

(30) Priority: 13.01.2025 US 202563744652 P
(71) Applicant: Flexicare (Group) Limited, Mountain Ash, Mid Glamorgan CF45 4ER (GB)
(72) Inventor: POORMAND, Ghassem, Mountain Ash, CF45 4ER (GB)
(74) Representative: Mewburn Ellis LLP

(57) **Abstract**

A fluid delivery system including a nasal cannula having a manifold is provided. The manifold includes a housing including an internal cavity, and a pair of nasal prongs extending from the housing. Each of the pair of nasal prongs include a first conduit extending from the housing and in flow communication with the internal cavity of the housing. The first conduit is configured to supply oxygen to a patient. Each of the pair of nasal prongs also include a second conduit positioned adjacent the first conduit, where the second conduit is configured to receive carbon dioxide (CO₂) exhaled by the patient, and an internal wall separating the first conduit and the second conduit.

## Description

### CROSS-REFERENCE TO RELATED APPLICATIONS

This application claims the benefit of U.S. Provisional Patent Application No. 63/744,652, filed January 13, 2025, which is incorporated herein by reference it its entirety.

### TECHNICAL FIELD

The present disclosure relates generally to fluid delivery systems including nasal cannulas, and more particularly, to nasal cannulas including manifolds having nasal prongs.

### BACKGROUND

Nasal cannulas have been widely used in medical settings to deliver supplemental oxygen or increased airflow to patients in need of respiratory support. Traditional nasal cannula designs typically consist of a pair of prongs that are inserted into the nostrils, connected to a single conduit that supplies the necessary airflow or oxygen. These designs have been effective in providing basic respiratory assistance; however, they often lack the ability to deliver precise and controlled airflow to each nostril independently, which can be crucial for certain medical conditions or treatments.

Previous approaches to improving nasal cannulas have focused on enhancing patient comfort and optimizing airflow delivery. Some designs have incorporated softer materials for the prongs to reduce irritation and improve fit within the nostrils. Others have experimented with varying the shape and size of the prongs to better accommodate different nasal anatomies. Additionally, some advancements have been made in the materials used for the conduits to ensure flexibility and durability while maintaining comfort and a consistent flow of oxygen or air.

Efforts have also been made to address the issue of airflow distribution by introducing dual-lumen designs, where each prong is connected to a separate conduit. This allows for more controlled and balanced delivery of airflow to each nostril. However, these designs often involve complex configurations and can be cumbersome to manufacture and use.

In addition to supplying oxygen to patients, it is often crucial to monitor the breathing of the patient receiving the oxygen supplied by nasal cannula. Common practices include additional, distinct tubes that are positioned near a patient's nose and/or mouth - often directly adjacent to nasal cannulas - which can receive carbon dioxide (CO₂) exhaled by the patient. However, these distinct tubes can be cumbersome, take up additional space around a patient's nose/mouth, and frequently get pushed aside due to patient movement and/or in favour of keeping the nasal cannula in place for delivering oxygen. As a result, the monitoring of exhaled CO₂ by the patient is often inaccurate, rendering conventional monitoring tubes impractical and superfluous.

### SUMMARY

An aspect of the present disclosure is directed to a manifold for a nasal cannula. The manifold includes a housing including an internal cavity, and a pair of nasal prongs extending from the housing. Each of the pair of nasal prongs include a first conduit extending from the housing and in flow communication with the internal cavity of the housing. The first conduit is configured to supply oxygen to a patient. Each of the pair of nasal prongs also include a second conduit positioned adjacent the first conduit, where the second conduit is configured to receive carbon dioxide (CO₂) exhaled by the patient, and an internal wall separating the first conduit and the second conduit.

Another aspect of the present disclosure is directed to an assembly including a gaseous supply tube and a nasal cannula including a manifold coupled to the gaseous supply tube. The manifold includes a housing including an internal cavity, and a pair of nasal prongs extending from the housing. Each of the pair of nasal prongs include a first conduit extending from the housing and in flow communication with the internal cavity of the housing. The first conduit is configured to supply oxygen to a patient. Each of the pair of nasal prongs also include a second conduit positioned adjacent the first conduit, where the second conduit is configured to receive carbon dioxide (CO₂) exhaled by the patient, and an internal wall separating the first conduit and the second conduit.

An additional aspect of the present disclosure is directed to a system includes an assembly including a gaseous supply tube and a nasal cannula including a manifold coupled to the gaseous supply tube. The manifold includes a housing including an internal cavity, and a pair of nasal prongs extending from the housing. Each of the pair of nasal prongs include a first conduit extending from the housing and in flow communication with the internal cavity of the housing. Each of the pair of nasal prongs also include a second conduit positioned adjacent the first conduit, and an internal wall separating the first conduit and the second conduit. The system also includes a connector coupled to the housing of the manifold and in flow communication with the second conduit of each of the pair of nasal prongs. Additionally, the system includes a line coupled to and extending between the connector and a bite block.

### BRIEF DESCRIPTION OF THE DRAWINGS

Embodiments of the present disclosure will now be described with reference to the accompanying drawings, in which:
Figure 1 shows a perspective view of a fluid delivery system including a nasal cannula according to a first example;
Figure 2 shows a perspective view of a portion of the system of Figure 1 including a bite block;
Figure 3 shows a front perspective view of a manifold of the nasal cannula for the system of Figure 1;
Figure 4 shows a rear perspective view of the manifold of the nasal cannula for the system of Figure 1;
Figure 5 shows a rear view of a housing of the manifold for the nasal cannula;
Figure 6 shows a side cross-section view of the housing of the manifold for the nasal cannula of Figure 5;
Figure 7 is a perspective rear view of a manifold of a nasal cannula for a fluid delivery system according to a second example;
Figure 8 is an enlarged, perspective rear view of the manifold of the nasal cannula of Figure 7; and
Figure 9 shows a rear perspective view of another embodiment of a manifold of the nasal cannula for the system of Figure 1.

It is noted that the drawings of the disclosure are not to scale. The drawings are intended to depict only typical aspects of the disclosure, and therefore should not be considered as limiting the scope of the disclosure. In the drawings, like numbering represents like elements between the drawings.

### DETAILED DESCRIPTION

As discussed herein, the disclosure relates generally to generally to fluid delivery systems including nasal cannulas, and more particularly, to nasal cannulas including manifolds having nasal prongs.

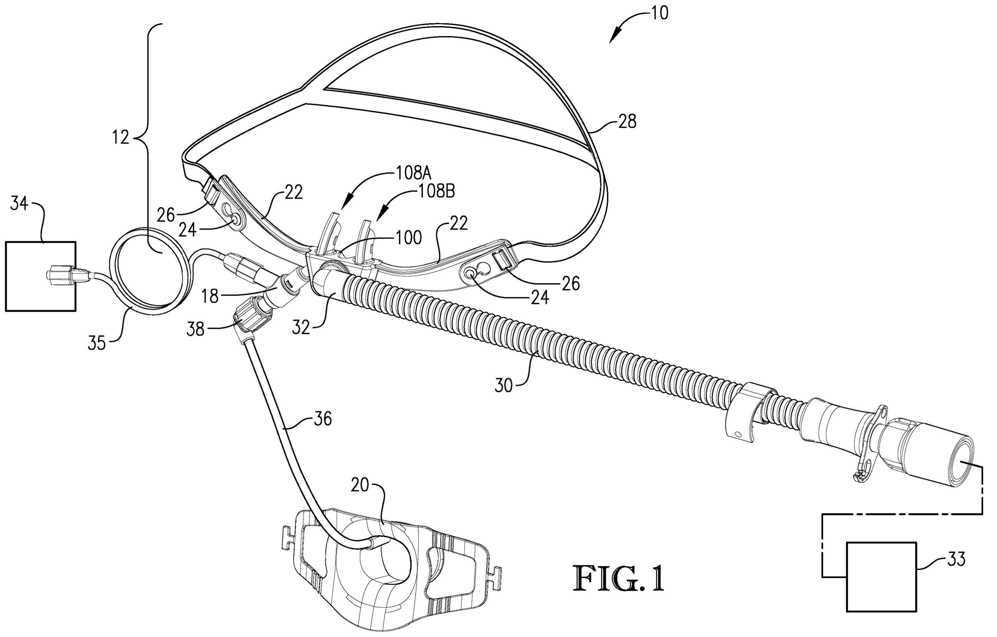

These and other embodiments are discussed below with reference to FIGS. 1-8. However, those skilled in the art will readily appreciate that the detailed description given herein with respect to these Figures is for explanatory purposes only and should not be construed as limiting.

FIG. 1 shows a fluid delivery system 10 (hereafter, "system 10") that facilitates the delivery of gaseous fluids (e.g., oxygen) to a patient, as well as detect and/or monitor carbon dioxide (CO₂) being expelled or exhaled from the patient. In the non-limiting example, system 10 includes a nasal cannula 12, a connector 18 of a carbon dioxide (CO₂) monitoring device coupled to and/or in communication with nasal cannula 12, and a bite block 20 coupled to connector 18 of CO₂ monitoring device. In non-limiting examples, nasal cannula 12 is used for high flow oxygen therapy (HFOT), which uses flow rates many times higher than those used typically with face masks and standard nasal cannulas. For instance, the flow rates to a mask or standard cannula may be approximately 5 Standard litres per minute (LPM) whilst nasal cannula 12 of system 10 may provide flow rates of between 30 LPM and 70 LPM or higher, in adults and 11-20 LPM in neonates thereby creating a continuous low level of (3-5cm H2O) positive airway pressure effect in nasopharynx so that a greater area of the patent's lungs is recruited for gas exchange, further improving blood oxygenation.

As discussed herein, nasal cannula 12 includes a manifold 100 arranged to rest against a patient's face. Additionally, and as discussed herein, a pair of non-sealing nasal prongs extend from manifold 100, which each enter a respective nasal passage (nare) of the patient to facilitate the delivery of a fluid to the patient, as well as receiving CO₂ exhaled by the patient.

A pair of opposing straps 22 extend from manifold 100. Straps 22 are arranged to extend around part of the patients face and include end protrusions 24 at a distal end. The end protrusions 24 provide a connection point to which corresponding clips 26 can be attached for clipping to a patient head band 28, such that different sizes and lengths of head band 28 can be selected for a given patient or an adjustable head band can be provided. Straps 22 may take any suitable form to secure the nasal cannula 12 to the patient, for example a single strap may extend from each side of the manifold 100 to create a loop that can be fitted over a patient's head.

A gaseous supply tube 30 connects to manifold 100 via a supply connector 32. Supply connector 32 is received inside an opening 104 of manifold 100 to form the connection between the components, as discussed herein. Supply connector 32 may be an elbow connector that redirects the flow such that the gaseous fluid flows through opening 104 and is angled with respect to the gas flow through gaseous supply tube 30 adjacent to supply connector 32. Supply connector 32 provides a sharp right angle where two respective perpendicular sections of the connector meet, although in alternative examples supply connector 32 may be swept and/or curved such that angular change is more gradual and there is no sudden angle change, thereby decreasing any pressure drop.

Gaseous supply tube 30 connected to supply connector 32 may be flexible. Gaseous supply tube 30 may also be sufficiently resilient to retain a substantially constant cross section of air/gas flow yet can bend so the gas tube can be comfortably routed to a patient's face. Additionally, gaseous supply tube 30 may be a corrugated tube, (e.g., with a corrugated outer surface). The inner surface of gaseous supply tube 30 may also be corrugated, although in alternative examples the inner and/or outer surface may be smooth. In non-limiting examples, gaseous supply tube 30 is fluidly coupled to and/or in flow communication with at least one supply device 33 of system 10. Supply device 33 is positioned upstream of gaseous supply tube 30 and nasal cannula 12, respectively. Supply device 33 is formed as any suitable device, component, and/or system configured to supply a gas (e.g., oxygen) to nasal cannula 12 during operation of system 10. For example, supply device 33 may include a humidifier, a blower/fan, and/or an oxygen (O₂) supply (e.g., oxygen tank, wall connection, etc.).

Connector 18 of system 10 is coupled to and/or in communication with manifold 100 and CO₂ monitoring device 34, via interconnect 35. As discussed herein, CO₂ monitoring device 34 facilitates the sampling, monitoring, or detection of CO₂ exhaled by the patient wearing nasal cannula 12. Additionally, as discussed herein, connector 18 is configured to receive CO₂ from manifold 100 and subsequently provide the CO₂ and/or a signal to CO₂ monitoring device 34 via interconnect 35 coupled directly to and/or in communication with both connector 18 and CO₂ monitoring device 34, respectively. In non-limiting examples, interconnect 35 is formed from any suitable material that is configured to provide CO₂ exhaled from a patient from connector 18 of system 10 to CO₂ monitoring device 34. CO₂ monitoring device 34 is formed as any suitable device, system, and/or apparatus (e.g., computing device) that is configured to receive CO₂ from connector 18 (e.g., via interconnect 35) and/or a CO₂ detection signal from connector 18 to determine the patient wearing system 10 including manifold 100 is breathing desirably and/or processing the oxygen supplied via system 10.

Connector 18 of system 10 is also coupled to a line 36 of system 10. More specifically, line 36 extends between connector 18 coupled to manifold 100 and a bite block 20 of system 10. Line 36 is coupled to connector 18 via coupling component 38. That is, a first end of line 36 is coupled directly to coupling component 38, which in turn is coupled directly to connector 18. Coupling component 38 facilitates the fluid coupling between line 36. As discussed herein, a second end of line 36, opposite first end coupled to coupling component 38, is coupled to bite block 20 of system 10. Similar to manifold 100 and its various components discussed herein, line 36 provides CO₂ exhaled from a patient's mouth to connector 18 for additional sampling, monitoring, and/or detecting by CO₂ monitoring device 34. Line 36 is formed from any suitable material that is configured to provide CO₂ exhaled from a patient to connector 18 of system 10. In non-limiting examples where coupling component 38 and line 36, are removed from system 10 (e.g., post-surgery), connector 18 prevents air flow from entering connector 18 and/or mixing with CO₂ exhaled from the patient and provided to manifold 100, as discussed herein. For example, connector 18 includes a self-sealing valve that substantially seals connector 18 and prevents air from flowing into connector 18 when coupling component 38 is uncoupled and/or detached from connector 18 during operation of system 10.

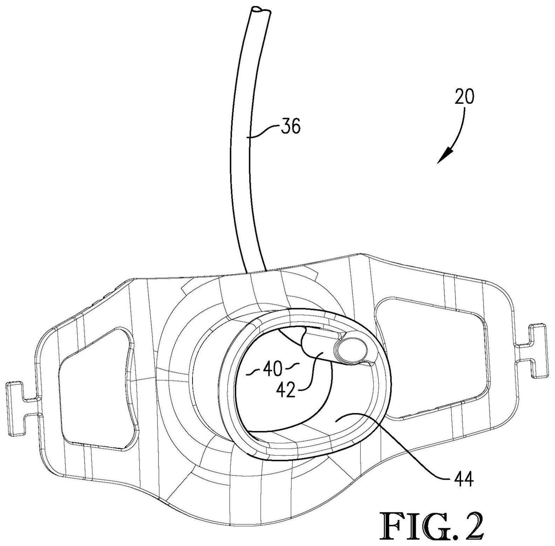

Line 36 is coupled to bite block 20 of system 10. That is, and as shown in FIG. 2, line 36 is coupled to and/or is at least partially disposed within a breathing orifice 40 (hereafter, "orifice 40") of bite block 20. In the non-limiting example, an interconnection 42 is formed in and/or on an inner surface 44 of orifice 40 of bite block 20. Interconnection 42 is configured to receive and/or be coupled to at least a portion of line 36 extending from and coupled to connector 18, such that line 36 is positioned within, directly adjacent to, and is in flow communication with orifice 40 of bite block 20. Orifice 40 of bite block 20 is configured to be positioned and/or extend into the patient's mouth. As such, and during operation, line 36 may be provided and/or receive a portion of CO₂ exhaled from the patient's mouth and through orifice 40.

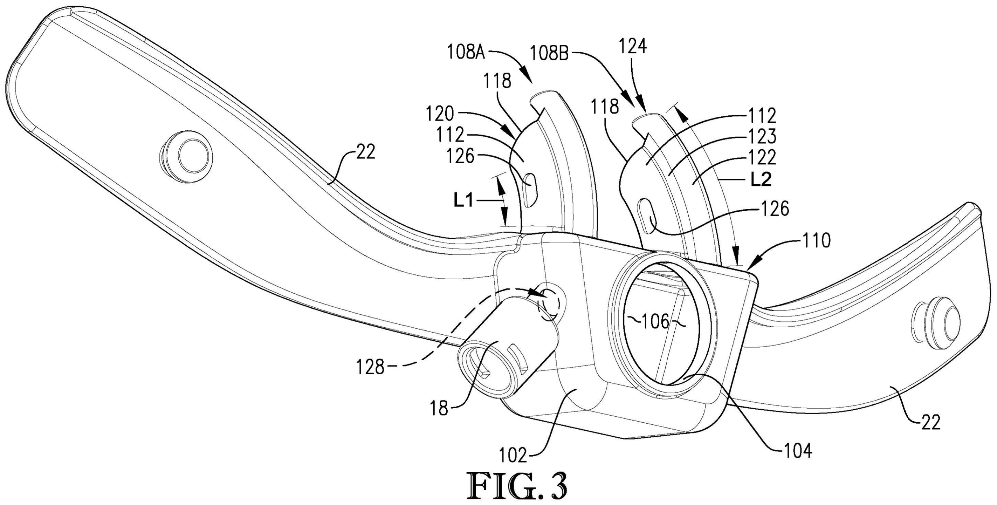

FIGS. 3-5 show various views of manifold 100 for cannula 12 of system 10. Manifold 100 includes housing 102 and opening 104 formed there through. Opening 104 formed through housing 102 may be centrally located on manifold 100, such that opening 104 is located equidistant between nasal prongs of manifold 100, as discussed herein. In alternative examples, opening 104 can be offset such that it is further from one of the nasal prongs of manifold 100. Opening 104 may be a single inlet aperture fluidically connecting the gaseous supply tube 30 to manifold 100. That is, opening 104 is configured to receive and/or be coupled to supply connector 32 coupled to gaseous supply tube 30, such that during operation, gaseous supply tube 30 can provide fluid (e.g., oxygen) to manifold 100.

Housing 102 of manifold 100 also defines internal cavity 106. Internal cavity 106 is in flow communication with opening 104 formed through housing 102 and is configured to receive the fluid supplied to manifold 100 via gaseous supply tube 30. As discussed herein, internal cavity 106 is also fluidly coupled to and/or in flow communication with nasal prongs of manifold 100 for supplying the gaseous fluid (e.g., oxygen) to the nasal prongs during operation.

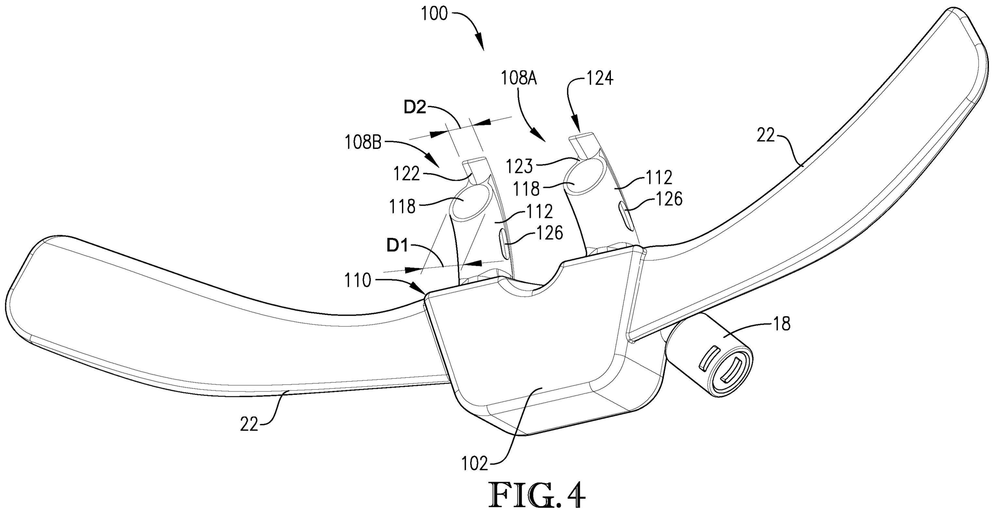

Manifold 100 also include pair of nasal prongs 108A, 108B extending from housing 102. For example, and as shown in FIGS. 3 and 4, each of the pair of nasal prongs 108A, 108B extend from a top or upper section 110 of housing 102. Each nasal prong 108A, 108B is formed as single entity component including a plurality of features. That is, nasal prongs 108A, 108B of manifold 100 are each formed as a single, integral component including a plurality of distinct portions and/or features discussed herein. In the non-limiting example, each nasal prong 108A, 108B includes a single sidewall 112 including a plurality of features extending therethrough. In other non-limiting examples, each feature or component (e.g., conduits) of nasal prongs 108A, 108B may be formed as distinct features that are coupled to one another to form nasal prongs 108A, 108B of manifold 100.

Nasal prongs 108A, 108B include a first conduit 118 extending from housing 102. More specifically, first conduit 118 extends outward from upper section 110 of housing 102. First conduit 118 is also in flow communication with internal cavity 106 of housing 102. During operation, gaseous fluid provided to manifold 100 via gaseous supply tube 30 flows through internal cavity 106 of housing 102 for manifold 100 and into and/or through first conduit 118 of nasal prongs 108A, 108B. First conduit 118 includes first length (L1) measured between upper section 110 of housing 102 and a distal end 120 of first conduit 118. Additionally, and as shown in FIG. 4, first conduit 118 includes a diameter (D1).

Nasal prongs 108A, 108B also include a second conduit 122. Similar to first conduit 118, second conduit 122 of each nasal prong 108A, 108B extends from housing 102, and more specifically from upper section 110 of housing 102. Second conduit 122 is formed adjacent first conduit 118 in each nasal prong 108A, 108B. In non-limiting examples, an internal wall 123 extends between and/or separates first conduit 118 and second conduit 122 in each nasal prongs 108A, 108B of manifold 100. Second conduit 122 of prongs 108A, 108B includes a second length (L2) measured between upper section 110 of housing 102 and a distal end 124 of second conduit 122. Second length (L2) of second conduit 122 is greater than first length (L1) of first conduit 118. As such, and when positioned within a patient's nasal cavity or passageway, distal end 124 of second conduit 122 extends beyond and/or further into patient's nasal passageway than distal end 120 of first conduit 118. Additionally, second conduit 122 includes a second diameter (D2). In non-limiting examples, second diameter (D2) is smaller than first diameter (D1) of first conduit 118. As discussed herein, second conduit 122 of each of the pair of nasal prongs 108A, 108B may be in fluid communication with connector 18 of system 10 (see, FIG. 1) for supplying CO₂ received in second conduit 122 to connector 18.

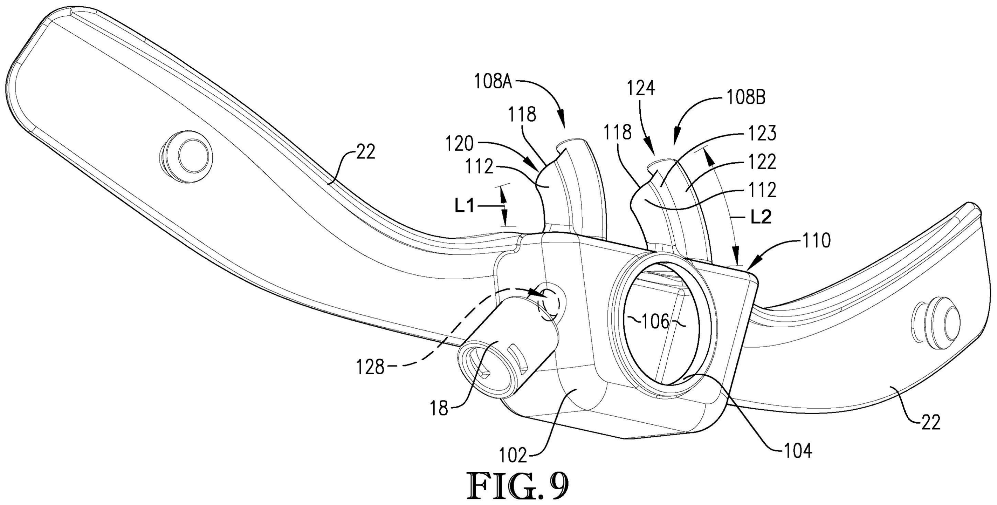

As shown in FIG. 9, in some embodiments, the first length (L1) of the first conduit 118 and the second length (LW) of the second conduit 122 are relatively shorter than the first and second lengths (L1, L2) of the embodiment of FIG. 4.

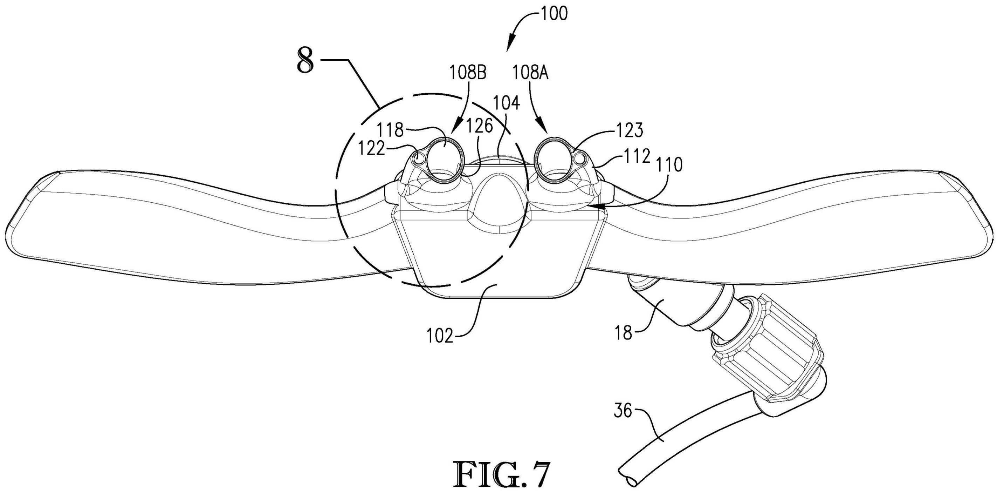

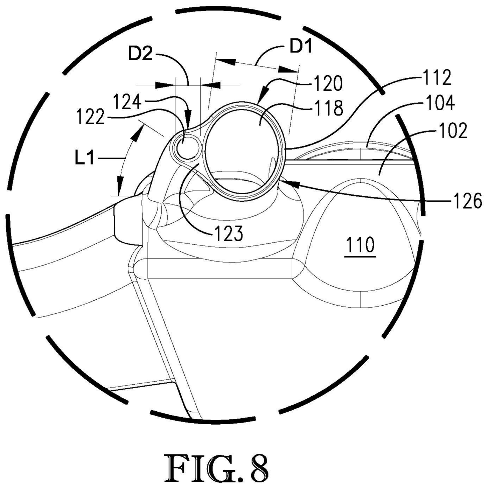

Second conduit 122 is positioned radially outward from first conduit 118. In non-limiting example shown in FIGS. 3 and 4, second conduit 122 is radially outward from and radially above (e.g., substantially in a "12-o'clock position") first conduit 118 in each of the pair of nasal prongs 108A, 108B. In other non-limiting examples (see, FIGS. 7 and 8), second conduit 122 is radially outward from and/or on a side of first conduit 118.

Each nasal prong 108A, 108B also includes at least one vent hole 126 formed therethrough. In non-limiting example shown in FIGS. 3 and 4, each nasal prong 108A, 108B includes two vent holes 126 extending through sidewall 112 of nasal prongs 108A, 108B, radially opposite one another and radially aligned with first conduit 118. Additionally, each of the two vent holes 126 are formed in nasal prongs 108A, 108B between housing 102 and distal end 120 of first conduit 118. Vent holes 126 are in flow communication with first conduit 118 and/or extend through sidewall 112 of 108A, 108B to form a substantially passageway that extends substantially perpendicular to each first conduit 118 of nasal prongs 108A, 108B. During operation, vent holes 126 allow oxygen or fresh air to escape first conduit 118 when the patient exhales. Additionally, vent holes 126 allow CO₂ exhaled by the patient to flow through second conduits 122 without the risk of mixing with fresh gas or oxygen provided by first conduit 118. That is, oxygen or gas supplied by first conduit 118 is expelled through vent holes 126 as a result of the pressure applied by and/or fluid flow of exhaled air from the patient. The exhaled CO₂ is provided to and/or flows through second conduits 122. Allowing oxygen or fresh air to be expelled from vent holes 126 minimizes or eliminates the risk of the supplied oxygen or fresh air from flowing from the distal end 120 of first conduit 118 directly into the second conduit 122. Additionally, or alternatively, the inclusion of vent holes 126 within first conduit 118 reduces or eliminates undesirable back pressure, back flow, and/or flow blockage within first conduit 118 and/or housing 102 of manifold 100 when a patient exhales.

Although each nasal prong 108A, 108B includes two distinct vent holes 126, it is to be understood that each nasal prong 108, 108B can include more or less vent holes 126. Additionally, it is to be understood that the position of each vent hole(s) 126 formed through first conduit 118 of nasal prong 108A, 108B can vary. For example, each nasal prong 108A, 108B can include a single vent hole 126 formed through sidewall 112, radially opposite second conduit 122 (see, FIGS. 7 and 8). In some embodiments, the vent holes 126 are eliminated (FIG. 9).

Housing 102 of manifold 100 may also include aperture 128 formed therethrough. More specifically, and as shown in FIG. 3, aperture 128 extends through housing 102 and is configured to receive and/or be coupled to connector 18 for CO₂ monitoring device 34. In the non-limiting example, aperture 128 is formed through housing 102 of manifold 100 adjacent to opening 104.

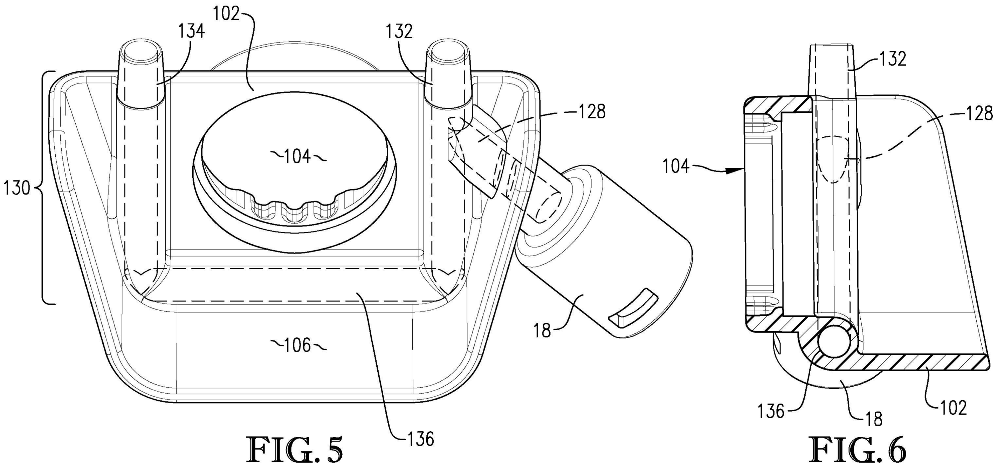

Housing 102 of manifold 100 also includes internal channel 130 positioned therein. In non-limiting examples shown in FIGS. 5 and 6, internal channel 130 is positioned and/or formed within housing 102 and extends between aperture 128 formed through housing 102 and second conduit 122 of each of the pair of nasal prongs 108A, 108B. Additionally, internal channel 130 is in flow communication with and/or fluidly couples aperture 128 and second conduit 122 of each of the pair of nasal prongs 108A, 108B.

As shown in FIGS. 5 and 6, internal channel 130 includes first portion 132 in flow communication with second conduit 122 of first nasal prong 108A. First portion 132 of internal channel 130 is also in direct flow communication with aperture 128. More specifically, aperture 128 formed through housing 102 is in direct flow communication with and/or is formed directly adjacent first portion 132 of internal channel 130. First portion 132 of internal channel 130 also extends toward upper section 110 of housing 102 (see, FIG. 3) and second conduit 122 of first nasal prong 108A. During operation, CO₂ flows from the patient wearing nasal canula assembly 12, through second conduit 122 of first prong 108A to first portion 132 of internal channel 130. The patient expelled CO₂ subsequently flows to connector 18 via first portion 132 and aperture 128 for detection by CO₂ monitoring device 34, as discussed herein.

Internal channel 130 also include second portion 134 positioned within housing 102. Second portion 134 positioned and/or extending through housing 102 is in flow communication with second conduit 122 of second prong 108B. Additionally, second portion 134 of internal channel 130 also extends toward upper section 110 of housing 102 (see, FIG. 3) and second conduit 122 of second nasal prong 108B. Similar to first portion 132 and during operation, CO₂ flows from the patient, through second conduit 122 of second nasal prong 108B to second portion 134, and ultimately to connector 18 for detection by CO₂ monitoring device 34.

Intermediate portion 136 of internal channel 130 extends between first portion 132 and second portion 134. More specifically, intermediate portion 136 positioned within housing 102 extends between, fluidly couples, and/or is in flow communication with first portion 132 and second portion 134 of internal channel 130. In non-limiting examples shown in FIGS. 5 and 6, intermediate portion 136 extends between first portion 132 and second portion 134, adjacent opening 104 formed through housing 102. As a result of fluidly coupling second portion 134 to first portion 132, intermediate portion 136 facilitates the sharing of CO₂ from second portion 134 of internal channel 130 to first portion 132, and ultimately to CO₂ monitoring device 34 of system 10 via connector 18. That is, CO₂ received in second portion 134 via second conduit 122 of second nasal prong 108B is flowed to first portion 132 via intermediate portion 136. The patient exhaled CO₂ provided to first portion 132 of internal channel 130 flows to connector 18 via first portion 132 and aperture 128, respectively, for detection by CO₂ monitoring device 34, as discussed herein.

FIGS. 7 and 8 show another non-limiting example of manifold 100 for system 10. It is understood that similarly numbered and/or named components may function in a substantially similar fashion. Redundant explanation of these components has been omitted for clarity.

In the non-limiting example shown in FIGS. 7 and 8, each of the pair of nasal prongs 108A, 108B include first conduit 118 and second conduit 122, as similarly described herein. Second conduit 122 is positioned and/or formed radially outward from first conduit 118, and on outer sides of nasal prongs 108A, 108B (e.g., second conduit 122 substantially in "3 o'clock" position on first prong 108A, second conduit 122 substantially in "9 o'clock" position on second prong 108B).

As similarly discussed herein, first conduit 118 includes a first diameter (D1), while second conduit 122 includes a second diameter (D2) that is smaller or less than the first diameter (D1). In the non-limiting example shown in FIGS. 7 and 8, both first conduit 118 and second conduit 122 include a length (L). That is, both first conduit 118 and second conduit 122 include substantially similar lengths (L) measured between upper section 110 of housing 102 and respective distal ends 120, 124.

Additionally in the non-limiting example shown in FIGS. 7 and 8, each nasal prong 108A, 108B includes a single vent hole 126 extending therethrough. More specifically, each nasal prong 108A, 108B includes single vent hole 126 extending through sidewall 112 of nasal prongs 108A, 108B, radially opposite and/or radially aligned with second conduit 122, and/or through an inner side of sidewalls 112 for nasal prongs 108A, 108B. Additionally, vent hole 126 is formed in nasal prongs 108A, 108B between housing 102 and distal end 120 of first conduit 118. Vent holes 126 are in flow communication with first conduit 118 and/or extend through sidewall 112 of 108A, 108B to form a substantially passageway that extends substantially perpendicular to each first conduit 118 of nasal prongs 108A, 108B.

The foregoing drawings show some of the processing associated according to several embodiments of this disclosure. In this regard, each drawing or block within a flow diagram of the drawings represents a process associated with embodiments of the method described. It should also be noted that in some alternative implementations, the acts noted in the drawings or blocks may occur out of the order noted in the figure or, for example, may in fact be executed substantially concurrently or in the reverse order, depending upon the act involved. Also, one of ordinary skill in the art will recognize that additional blocks that describe the processing may be added.

Certain terminology of the present disclosure may refer to and describe relevant components within the disclosure. When doing this, common industry terminology may be used and employed in a manner consistent with its accepted meaning. Unless otherwise stated, such terminology should be given a broad interpretation consistent with the context of the present application and the scope of the appended claims. Those of ordinary skill in the art will appreciate that often a particular component may be referred to using several different or overlapping terms. What may be described herein as being a single part may include and be referenced in another context as consisting of multiple components. Alternatively, what may be described herein as including multiple components may be referred to elsewhere as a single part.

The terminology used herein is for the purpose of describing particular embodiments only and is not intended to be limiting of the disclosure. As used herein, the singular forms "a", "an" and "the" are intended to include the plural forms as well, unless the context clearly indicates otherwise. It will be further understood that the terms "comprises" and/or "comprising," when used in this specification, specify the presence of stated features, integers, steps, operations, elements, and/or components, but do not preclude the presence or addition of one or more other features, integers, steps, operations, elements, components, and/or groups thereof. "Optional" or "optionally" means that the subsequently described event or circumstance may or may not occur, and that the description includes instances where the event occurs and instances where it does not.

Approximating language, as used herein throughout the specification and claims, may be applied to modify any quantitative representation that could permissibly vary without resulting in a change in the basic function to which it is related. Accordingly, a value modified by a term or terms, such as "about," "approximately" and "substantially," are not to be limited to the precise value specified. In at least some instances, the approximating language may correspond to the precision of an instrument for measuring the value. Here and throughout the specification and claims, range limitations may be combined and/or interchanged, such ranges are identified and include all the sub-ranges contained therein unless context or language indicates otherwise. "Approximately" and/or "substantially" as applied to a particular value of a range applies to both values, and unless otherwise dependent on the precision of the instrument measuring the value, may indicate +/- 10% of the stated value(s).

The description of the present disclosure has been presented for purposes of illustration and description but is not intended to be exhaustive or limited to the disclosure in the form disclosed. Many modifications and variations will be apparent to those of ordinary skill in the art without departing from the scope and spirit of the disclosure. The embodiment was chosen and described in order to best explain the principles of the disclosure and the practical application, and to enable others of ordinary skill in the art to understand the disclosure for various embodiments with various modifications as are suited to the particular use contemplated.

## Claims

1. A manifold for a nasal cannula , the manifold comprising:
a housing including an internal cavity; and
a pair of nasal prongs extending from the housing, each of the pair of nasal prongs including:
a first conduit extending from the housing and in flow communication with the internal cavity of the housing, the first conduit configured to supply oxygen to a patient;
a second conduit positioned adjacent the first conduit, the second conduit configured to receive carbon dioxide (CO₂) exhaled by the patient; and
an internal wall separating the first conduit and the second conduit.

2. The manifold of claim 1, further comprising:
an aperture formed through the housing, a connector coupled to and in flow communication with the aperture; and
an internal channel positioned within the housing, the internal channel extending between and in flow communication with the second conduit of each of the pair of nasal prongs and the aperture.

3. The manifold of claim 2, wherein the internal channel includes:
a first portion in flow communication with the second conduit of a first nasal prong;
a second portion in flow communication with a second conduit of a second nasal prong; and
an intermediate portion extending between and in flow communication with the first portion and the second portion.

4. The manifold of any one of claims 1 to 3, wherein the second conduit for each of the pair of nasal prongs is positioned radially outward from the first conduit for each of the pair of nasal prongs.

5. The manifold of any one of claims 1 to 4, wherein the first conduit for each of the pair of nasal prongs includes a first length, and the second conduit for each of the pair of nasal prongs includes a second length, greater than the first length.

6. The manifold of any one of claims 1 to 5, wherein the first conduit for each of the pair of nasal prongs includes a first diameter, and the second conduit for each of the pair of nasal prongs includes a second diameter, less than the first diameter.

7. An assembly, comprising:
a gaseous supply tube; and
a nasal cannula including the manifold of any one of claims 1 to 6.

8. A system comprising:
the assembly of claim 7;
a connector coupled to the housing of the manifold and in flow communication with the second conduit of each of the pair of nasal prongs; and
a line coupled to and extending between the connector and a bite block.

9. The system of claim 8, further comprising:
a carbon dioxide (CO₂) monitoring device in communication with the connector, the CO₂ monitoring device configured to detect CO₂ supplied to the connector via at least one of:
the second conduit of at least one of the pair of nasal prongs, or
the line extending between the connector and the bite block; and
an interconnect coupled to and in communication with the connector and the CO₂ monitoring device.

10. The system of claim 8 or claim 9, wherein the connector includes a self-sealing valve included therein, the self-sealing valve configured to prevent air flow into the connector in response to the line being uncoupled from the connector.

11. The system of any one of claims 8 to 10, further comprising at least one supply device fluidly coupled to the gaseous supply tube, the at least one supply device including (i) a humidifier, (ii) a blower, or (iii) an oxygen supply.
